(19) **Europäisches Patentamt**
**European Patent Office**
**Office européen des brevets**

(11) **EP 1 913 875 B1**

(12) **EUROPEAN PATENT SPECIFICATION**

(45) Date of publication and mention
of the grant of the patent:
**12.01.2011 Bulletin 2011/02**

(51) Int Cl.:
***A61B 8/08*** *(2006.01)*

(21) Application number: **07020174.4**

(22) Date of filing: **16.10.2007**

(54) **Ultrasound system for fusing an ultrasound image and an external medical image**

Ultraschallsystem zur Fusion eines Ultraschallbildes und eines externen medizinischen Bildes

Système à ultrasons pour la fusion d'une image à ultrasons et image médicale externe

(84) Designated Contracting States:
**DE FR IT**

(30) Priority: **17.10.2006 KR 20060100910**

(43) Date of publication of application:
**23.04.2008 Bulletin 2008/17**

(73) Proprietor: **MEDISON CO., LTD.**
**Kangwon-do 250-870 (KR)**

(72) Inventors:
• **Kim, Cheol An**
**Gangnam-gu**
**Seoul 135-280 (KR)**

• **Shin, Seong Chul**
**Gangnam-gu**
**Seoul 135-280 (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(56) References cited:
**EP-A- 1 795 130**      **WO-A-01/06924**
**WO-A-2006/008300**      **US-A1- 2001 007 919**
**US-A1- 2005 033 160**      **US-B1- 6 546 279**

## Description

### 1. Field

The present invention generally relates to ultrasound diagnostic systems, and more particularly to an ultrasound system for displaying a medical needle in a fusion image of an ultrasound image and an external medical image.

### 2. Background

Surgical treatment using a medical needle such as ablator or biopsy has recently become popular due to relatively small incisions made in such a procedure. The surgical treatment is performed by inserting the medical needle into an internal region of a human body while referring to an internal image of the human body. Such surgical treatment, which is performed while observing internal organs of the human body with aid of a diagnostic imaging system, is referred to as an interventional treatment. The interventional treatment is performed by directing the medical needle to the lesion to be treated or examined through a skin with reference to images during the treatment. The images are acquired by employing a computerized tomography (CT) scanner generally used in a radiology department or a magnetic resonance imaging (MRI) system. Compared to a normal surgical treatment requiring relatively wide incisions to open the lesion, the interventional treatment has the advantages of low costs and obtaining effective operation results. This is because general anesthesia is not necessary for the interventional treatment and patients are subjected to less pain while benefiting from rapid recovery.

However, it is difficult to obtain such images in real time by using the CT scanner or the MRI system. Especially, when the interventional treatment is performed by using the CT scanner, both the patient and the operator are exposed to radiation for quite a long time. In contrast, when the interventional treatment is performed by using an ultrasound diagnostic system, the images can be obtained in real time while not affecting the human body. However, there is a problem in that it is difficult to accurately recognize the lesion in the ultrasound image obtained by using the ultrasound diagnostic system.

US 2005/033160 A1 discloses an image processing/displaying apparatus comprising a data storage unit configured to store 3-dimensional volume data, a cross section information generation unit configured to generate cross section information by reconstructing the 3-dimensional volume data stored in the data storage unit, an image display unit configured to display a cross section image of a subject in accordance with the cross section information generated by the cross section information generation unit, a locking unit configured to designate an affected part in the cross section image as a rotation center and a control unit configured to control image rotation on the image display unit so that the affected part is contained in the cross section image, when operation for rotating cross section is performed by an operator. Furthermore, a paracentesis needle insertion path, which is a virtual between the probe insertion point and the affected part center, is drawn by a probe position identification unit. By rotating the locked plane cut passing through the affected part center, the user determines whether the paracentesis needle insertion path is sufficiently apart from any blood vessel or the like. However, the distance between the lesion and the medical needle is not computed. US 6,546,279 B describes a computer controlled system for guiding the needle device, such as a biopsy needle, by reference to a single mode medical imaging system employing any one of computed tomography imaging (CTI) equipment, magnetic resonance imaging equipment (MRI), fluoroscopic imaging equipment, or 3D ultrasound system, or alternatively, by reference to a multi-modal imaging system, which includes any combination of the aforementioned systems. WO 2006/008300 A concerns an apparatus for fusion and navigation of ecographic and volumetric images of a patient and for localization of an ecographic probe connected with an ecographer and/or of a surgical instrument operating on the same patient.

### BRIEF DESCRIPTION OF THE DRAWINGS

Arrangements and embodiments may be described in detail with reference to the following drawings in which like reference numerals refer to like elements and wherein:

FIG. 1 is a block diagram showing an ultrasound system constructed according to one embodiment of the present invention;

FIG. 2 is schematic diagram showing an example of an external image of a target object attaching a position marker;

FIG. 3 is a photo for explaining designation of lesion positions on an external image;

FIGS. 4 to 6 are block diagrams showing ultrasound systems constructed according to embodiments of the present invention;

FIG. 7 is a block diagram showing a probe position information providing unit in accordance with one embodiment of the present invention;

FIG. 8 is a block diagram showing a medical needle position information providing unit in accordance with one embodiment of the present invention;

FIG. 9 is a schematic diagram for explaining a guide line input by a user in accordance with one embodiment of the present invention;

FIG. 10 is a schematic diagram showing a guide line formed between a lesion and a medical needle; and

FIG. 11 is a block diagram showing an image processing unit in the ultrasound system in accordance with one embodiment of the present invention.

**DETAILED DESCRIPTION**

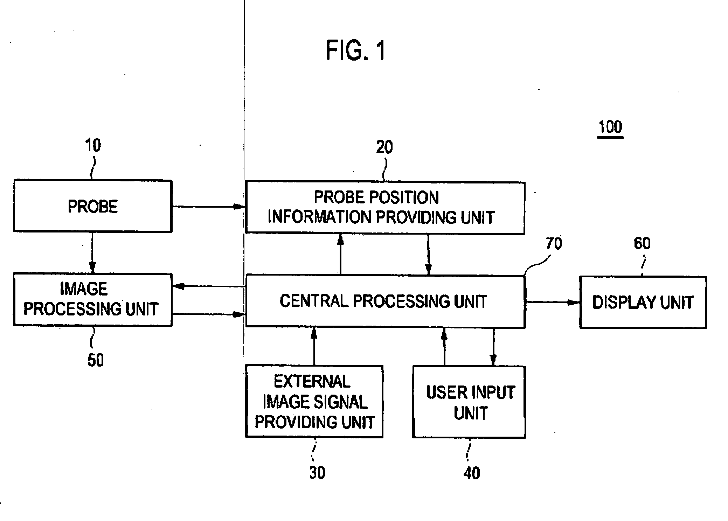

FIG. 1 is a block diagram showing an ultrasound system constructed according to one embodiment of the present invention. As shown in FIG. 1, the ultrasound system 100 includes a probe 10, a probe position information providing unit 20, an external image signal providing unit 30, a user input unit 40, an image processing unit 50, a display unit 60 and a central processing unit 70. The probe transmits ultrasound signals to a target object and receives ultrasound signals reflected from a lesion and a medical needle in the target object. The probe position information providing unit 20 provides position information of the probe on the target object. The position information of the probe includes information upon a direction of an ultrasound beam transmitted into the target object.

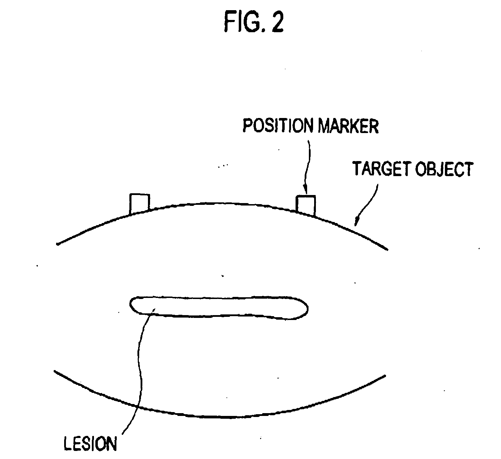

The external image signal providing unit 30 provides external image signals acquired from the external image device. The external image signals may be provided from a computerized tomography (CT) scanner, a magnetic resonance imaging (MRI) system or a positron emission tomography (PET) scanner. An external image formed based on the external image signals may show a target object and a medical needle inserted into the target object. The external image signals may be provided in a digital imaging communication format such as the digital imaging communication in medicine (DICOM) standard format. Also, the external image shows a lesion in the target object and a lesion position marker. For example, the external image is acquired while at leat one lesion position marker is attached on a surface of the target object as shown in FIG. 2. The position marker may be any type of substances, which are capable of distinguishing from the target object in the CT, MRI or PET image.

The user input unit 40 may be a mouse, a keyboard, a track ball or the like. The user input unit 40 receives position information of the lesion in the external image from a user. Further, the user input 40 receives a selection of fusion conditions of the ultrasound image and the external image.

The image processing unit 50 forms an ultrasound image based on the ultrasound echo signals and a fusion image of the ultrasound image and the external image based on the position information of the probe and the position information of the lesion. As mentioned above, if the fusion condition is inputted through the user input unit 40, then the image processing unit 50 forms the fusion image by reflecting the inputted fusion condition.

The display unit 60 displays at least one image of the ultrasound image and the fusion image formed in the image processing unit and the external image. The display unit 60 may also display at least two images of the ultrasound image, the external image and the fusion image in parallel.

The central processing unit 70 controls operations of the probe position information providing unit 20, the external image signal providing unit 30, the user input unit 40, the image processing unit 50 and the display unit 60. The central processing unit 70 may control input/output of the probe position information and the external image signals. The central processing unit 70 may further control input/output of the lesion position information between the image processing unit 50 and each of the probe position information providing unit 20, the external image signal providing unit 30 and the user input unit 40. The central processing unit 70 may process information or signals according to necessity.

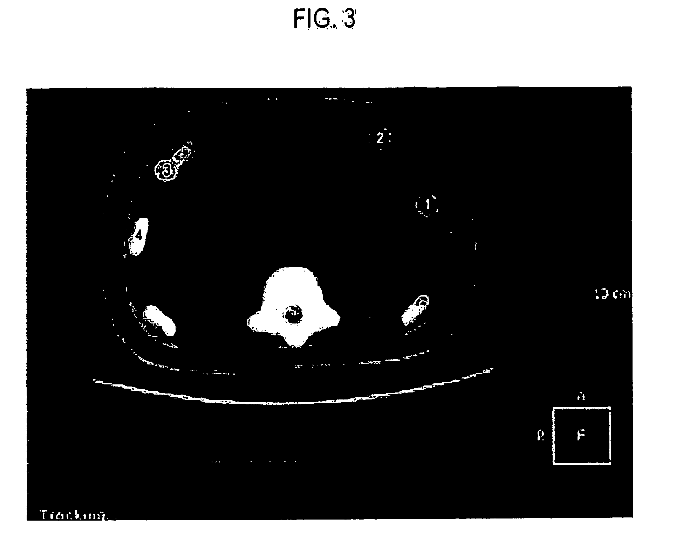

Hereinafter, a method for designating the lesion positions by the user will be described in detail with reference to FIG. 3. As shown in FIG. 3, while the external image showing the lesions and the lesion position markers is displayed on the display unit 60, the user designates positions of the lesions through mouse clicks or the like. The symbols 1, 2, 3 and 4 indicate the positions of 4 lesions designated on the external image through the mouse clicks by the user. In order to obtain a 3-dimensional position of a specific lesion, it is preferable that three or more positions of the specific lesion are designated in at least two external images containing the corresponding lesion. The position of the lesion, which is designated through the user input unit 40, that is, coordinates of the pixels corresponding to the lesion are inputted to the central processing unit 70.

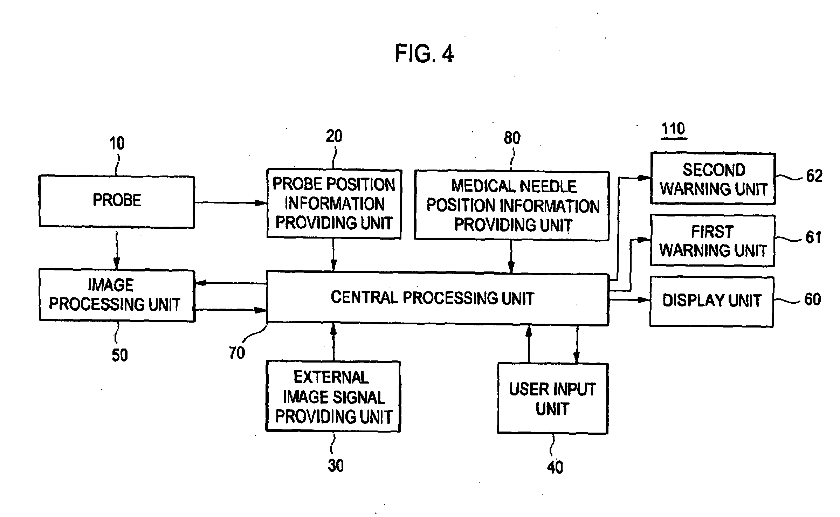

FIG. 4 is a block diagram showing an ultrasound system 110 in accordance with another embodiment of the present invention. The ultrasound system 110 further includes a medical needle position information providing unit 80 in addition to the elements of the ultrasound system 100 shown in FIG. 1. The medical needle position information providing unit 80 provides position information of a medical needle, which is inserted into the target object. The medical needle may be a biopsy needle or an ablator needle.

The central processing unit 70 in the ultrasound system 110 controls input/output of information between the display unit 60 and the medical needle position information providing unit 80. Further, central processing unit 70 processes the position information of the medical needle according to the necessity. The display unit 60 displays the position of the medical needle on the fusion image under the control of the central processing unit 70.

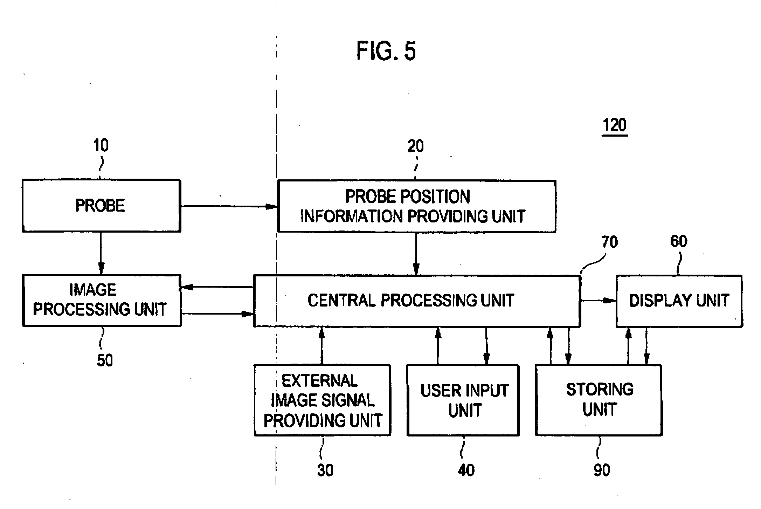

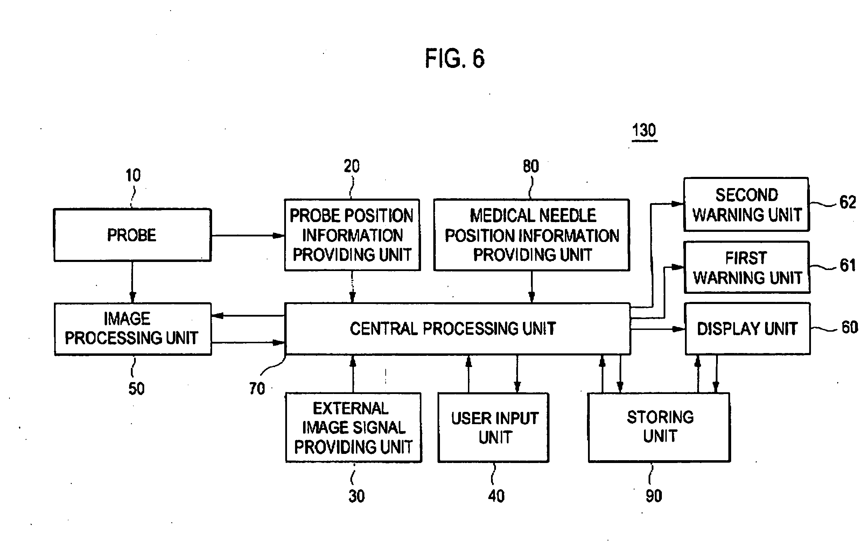

FIG. 5 is a block diagram showing an ultrasound system 120 in accordance with further another embodiment of the present invention. The ultrasound system 120 further includes a storing unit 90 in addition to elements of the ultrasound system 110. FIG. 6 is a block diagram showing an ultrasound system 130 in accordance with still another embodiment of the present invention. The ultrasound system 130 further includes a storing unit 90 in addition to elements of the ultrasound system 110 shown in FIG. 4. The storing unit 90 in the ultrasound systems 120 and 130 stores the fusion image formed in the image processing unit 50.

The user input unit 40 in each of the ultrasound systems 120 and 130, which are shown in FIGS. 5 and 6, receives a display screen save request from the user. The central processing unit 70 in each of the ultrasound systems 120 and 130 captures a screen, which is currently displayed on the display unit 60, in response to the display screen save request inputted from the user input 40 and stores the captured screen in the storing unit 90.

The central processing unit 70 in each of the ultrasound systems 110 and 130 computes a distance between the lesion and the medical needle based on the position information of the lesion inputted from the user input 40 and the position information of the medical needle inputted from the medical needle position information providing unit 80. The display unit 60 in each of the ultrasound systems 110 and 130 displays the distance between the lesion and the medical needle on the fusion image.

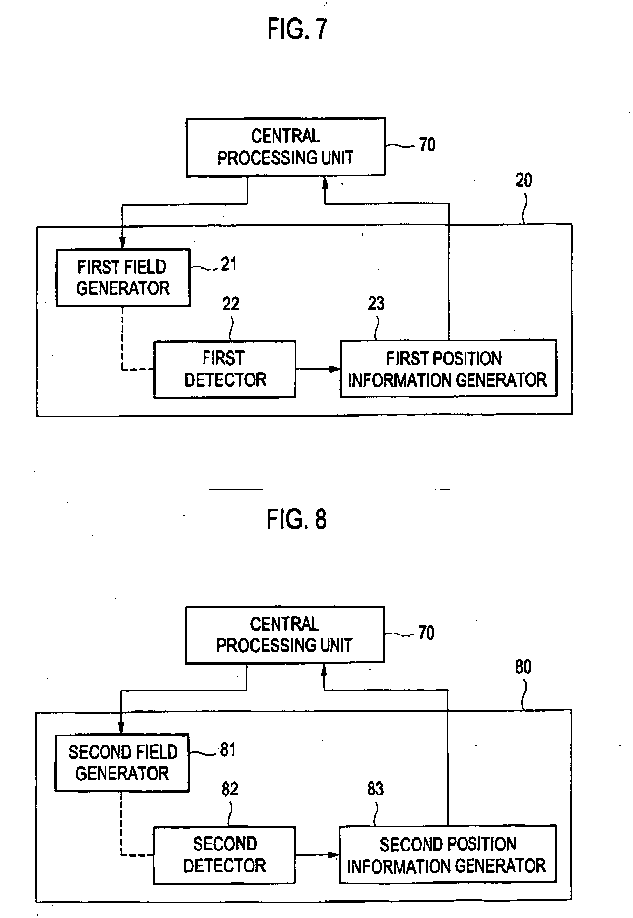

Referring to FIG. 7, the probe position information providing unit 20 in each of the ultrasound systems 100 to 130, which are shown in FIGS. 1, 4 and 6, includes a first field generator 21, a first detector 22 and a first position information generator 23. The first field generator 21 generates an electromagnetic field for tracking the position of the probe. The first position detector 22 may be mounted on a surface of the probe or built in the probe. The first position detector 22 generates a first detection signal in response to the electromagnetic field generated from the field generator 21. The position information generator 23 generates position information of the probe based on the first detection signal. The position detector 22 may be embodied with a coil sensor.

Referring to FIG. 8, the medical needle position information providing unit 80 in each of the ultrasound systems 110 and 130, includes a second field generator 81, a second detector 82 and a second position information generator 83, which is similar to the probe position information providing unit 20. The second field generator 81 generates an electromagnetic field, which may have a wavelength being capable of distinguishable from the electromagnetic field from the first field generator 21, for tracking the position of the medical needle. The second position detector 82 may be mounted on a surface of the medical needle or built in the medical needle. The second position detector 82 generates a second detection signal in response to the electromagnetic field generated from the second field generator 81. The position information generator 83 generates position information of the medical needle based on the second detection signal.

The probe position information providing unit 20 and the medical position information providing unit 80 in each of the ultrasound systems 110 and 130, may be embodied with a single position information providing unit. The position information providing unit may include a filed generator, a first detector, a second detector, a first position information generator and a second position information generator. The field generator generates an electromagnetic field for tracking the position of the probe and the position of the medical needle. The first position detector generates a first detection signal in response to the electromagnetic field. The second position detector generates a second detection signal in response to the electromagnetic field. The first position information generator generates position information of the probe based on the first detection signal. The second position information generator generates position information of the medical needle based on the second detection signal.

The user input unit 40 in each of the ultrasound systems 100 to 130, receives guide line information from the user. The central processing unit 70 in each of the ultrasound systems 100 to 130 generates position information of the guide line based on a trace TR of a cursor movable by the user with the mouse or the like or a plurality of points designated by the user with the mouse or the like on the fusion image. The display unit 60 displays the guide line GL on the fusion image FI based on the position information of the guide line GL.

The central processing unit 70 in each of ultrasound systems 110 to 130, may form position information of the guide line based on the position information of the lesion and the position information of the medical needle. The display unit 60 displays the fusion image inputted from the image processing unit 50 and the guide line on the fusion image based on the position information of the guide line.

The central processing unit 70 in each of ultrasound systems 110 to 130 compares the position information of the guide line and the position information of the medical needle to determine whether the medical needle deviates from the guide line. In such a case, each of the ultrasound systems 110 and 130 further includes a first warning unit 61 for notifying deviation of the medical needle under the control of the central processing unit 80. The first warning unit 61 may warn the deviation of the medical needle with sound or light.

The central processing unit 70 in each of the ultrasound systems 110 and 130, determines the time, at which the medical needle reaches the lesion, based on the position information of the lesion and the position information of the medical needle In such a case, each of the ultrasound systems 110 and 130 further includes a second warning unit 62 for notifying the arrival of the medical needle at the lesion under the control of the central processing unit 80. The second warning unit 62 may warn the arrival of the medical needle with sound or light. The first warning unit 61 and the second warning unit 62 may be embodied with one warning unit.

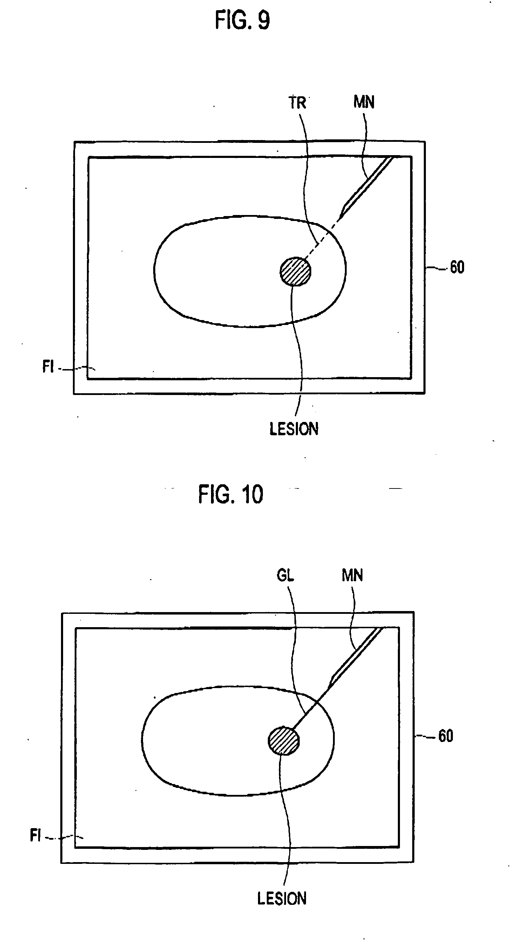

The image processing in each of the ultrasound systems 100 to 130 includes a first image processor 51, a second image processor 52 and a second image processor 53 as shown in FIG. 10. The first image processor 51 forms the ultrasound images based on the ultrasound echo signals inputted to the probe 10. The ultrasound images may include 2-dimensional ultrasound images, 3-dimensional ultrasound images and slice images. The second image processor 52 matches the coordinates of the external image with the coordinates representing probe positions based on the position information of the lesion in the external image inputted from the user and the position information of the probe, which is generated in the probe position information generating unit 20 so as to reconstruct the external image. The external image may be reconstructed to a 2-dimensional image, a 3-dimensional image or a slice image. The third image processor 53 fuses the ultrasound image and the reconstructed external image received from the first and second image processors 51 and 52, respectively. For example, a fused 2-dimensional image by be formed by fusing the 2-dimensional ultrasound image and the 2-dimensional external image or a fused slice image may be formed by fusing the ultrasound slice image and the external slice image. The third image processor 53 in each of the ultrasound systems 110 and 130 forms the fusion image, in which the position of the medical needle is indicated, based on the position information of the medical needle. In such a case, the display unit 60 displays the fusion image, in which the position of the medical needle is indicated, under the control of the central processing unit 70.

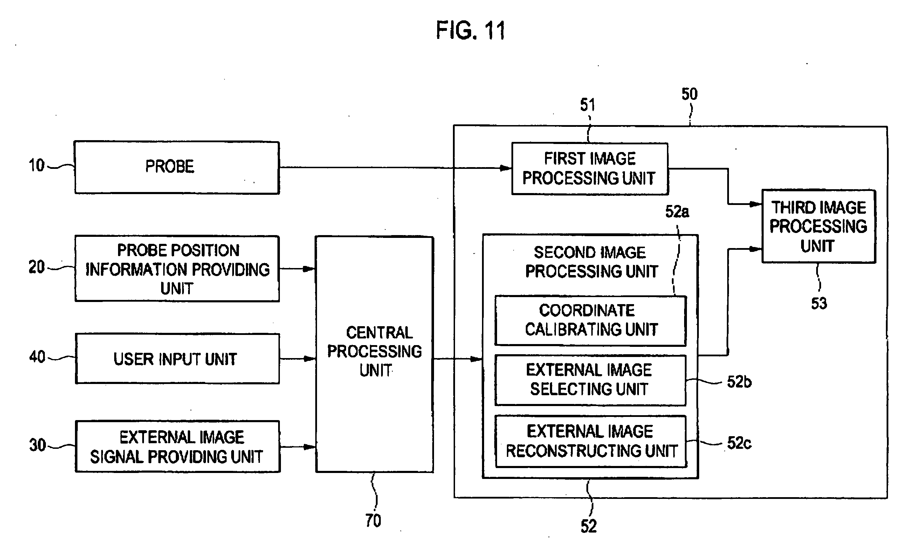

As shown in FIG. 11, the second image processor 52 includes a coordinate calibration unit 52a, an external image selection unit 52b and an external image reconstruction unit 52c. The coordinate calibration unit 52a calibrates coordinates of the lesion in the external image, which has different coordinates from coordinates of the ultrasound image. That is, the coordinate calibration unit 52a performs calibration upon origins in different coordinate systems including a coordinate system representing the external image such as the CT image, the MRI image or the PET image and a coordinate system representing the position of the probe, e.g., a global magnetic tracker coordinate system. For this calibration, the coordinate calibration unit 52a generates the coordinates of the lesion in the ultrasound image based on the position information of the probe inputted from the probe position information providing unit 20. The coordinate calibration unit 52a calibrates the coordinates of the lesion in the external image, which are inputted through the user input unit 40, based on the coordinates of the lesion in the ultrasound image. In case that the position of the lesion is designated by 4 points on each of two more external images, the coordinates of the lesion may be calibrated by using a 4-point matching method.

If the position of the lesion in the external image is expressed as position vectors g1, g2, g3 and g4, and the position of the lesion in the ultrasound image is expressed as position vectors v1, v2, v3 and v4, then the position vectors v1, v2, v3 and v4 may be considered as vectors obtained by applying a transform matrix M to the position vectors g1, g2, g3 and g4 as the following equation (1).

$$[v1\ v2\ v3\ v4] = M[g1\ g2\ g3\ g4] \qquad (1)$$

The transform matrix M is defined as the following equation (2).
$$M = [v1\ v2\ v3\ v4][g1\ g2\ g3\ g4]^{-1} \qquad (2)$$

As mentioned above, the coordinate calibration unit 52a applies the transform matrix M to the coordinates of the external image, thereby matching the coordinates of the external image with the coordinates of the ultrasound image.

The external image selection unit 52b selects an image, which is most similar to the ultrasound image among external images provided from external image signal providing unit 30, based on the coordinate calibration result.

The external image reconstruction unit 52c reconstructs the selected external image based on the coordinate calibration result. Thereafter, the reconstructed image may be rendered.

It is preferable that the ultrasound image and the external image may be fused in a voxel unit. The third image processor 53 may perform a minimum value-based fusing process, a maximum value-based fusing process or a weighted value-based fusing process according to the fusion condition inputted through the user input unit 40. A fusion voxel value

# EP 1 913 875 B1

Vf defined by a voxel value Vmc of the external image and a voxel value Vus of the ultrasound image according to the minimum value-based fusing process, the maximum value-based fusing process and the weighted value-based fusing process may be represented as the following equations (3), (4) and (5), respectively.

$$V_f(x,y,z) = Min(V_{mc}(x,y,z), V_{us}(x,y,z)) \qquad (3)$$

$$V_f(x,y,z) = Max(V_{mc}(x,y,z), V_{us}(x,y,z)) \qquad (4)$$

$$V_f(x,y,z) = \alpha \times (V_{mc}(x,y,z), (1-\alpha) \times V_{us}(x,y,z)) \qquad (5)$$

In the equation (5), a represents a weight value.

As mentioned above, since the fusion image of the ultrasound image and the external image is displayed in accordance with the present invention, the lesion in the target object can be more easily recognized. Therefore, it can provide convenience to an interventional ultrasound clinical application and reliability thereof can be improved.

An embodiment may be achieved in whole or in parts by the ultrasound system, including: a probe configured to be placed upon the target object and transmit ultrasound signals to the target object and receive ultrasound echo signals reflected from the target object, said target object including a lesion; a position information providing unit configured to provide position information of the probe on the target object; an external medical image signal providing unit configured to receive external medical image signals of the target object from an external imaging device; a user input unit configured to receive position information of the lesion in the external medical image from a user; an image processing unit configured to form an ultrasound image based on the ultrasound echo signals, form the external image based on the external image signals and form a fusion image of the ultrasound image and the external image based on the position information of the probe and the position information of the lesion; and a display unit configured to display the ultrasound image, the external image and the fusion image.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, and drawings.

In addition to variations and modifications in the component parts and/or arrangements, alternative uses will also be apparent to those skilled in the art.

## Claims

1. An ultrasound system (100, 110, 120, 130) comprising: a probe (10) configured to be placed upon the target object and transmit ultrasound signals to the target object and receive ultrasound echo signals reflected from the target object, said target object including a lesion; a position information providing unit (20) configured to provide position information of the probe on the target object; an external medical image signal providing unit (30) configured to receive external medical image signals of the target object from an external imaging device; a user input unit (40) configured to receive position information of the lesion in the external medical image from a user; an image processing unit (50) configured to form an ultrasound image based on the ultrasound echo signals, form the external image based on the external image signals and form a fusion image of the ultrasound image and the external image based on the position information of the probe and the position information of the lesion; a display unit (60) configured to display the ultrasound image, the external image and the fusion image; and a central processing unit (70) configured to control the position information providing unit (20), the external image signal providing unit (30), the user input unit (40), the image processing unit (50) and the display unit (60), wherein the position information providing unit (20,80) is further configured to provide position information of a medical needle inserted into the target object in the ultrasound image, and wherein the display unit (60) is adapted to display a position of the medical needle on the fusion image based on the position information of the medical needle under the control of the central processing unit, **characterized in that** the central processing unit (70) is adapted to compute a distance between the lesion and the medical needle based on the position information of the lesion and the position information of the medical needle, and the display unit (30) is adapted to display the distance between the lesion and the medical needle on the fusion image, wherein the position information providing unit (20,80) includes:

> a first field generator (21) configured to generate a first electromagnetic field to track the position of the probe;
> a first detector (22) mounted on or built in the probe for generating detection signals in response to the first electromagnetic field;
> a first position information generator (23) configured to generate the position information of the probe;
> a second field generator (81) configured to generate a second electromagnetic field to track the position of the medical needle;
> a second detector (82) mounted on or built in the medical needle configured to generate detection signals in response to the second electromagnetic field; and
> a second position information generator (83) configured to generate the position information of the medical needle.

2. The ultrasound system of Claim 1, further comprising a storing unit (90) configured to store the ultrasound image, the external image, the fusion image and an image displayed on display unit (60),
wherein the user input unit (40) is configured to further receive a display screen save request from the user, the central processing unit (70) is configured to capture a screen displayed on the display unit (60) in response to the display screen save request and the captured screen is stored in the storing unit (90).

3. The ultrasound system of Claim 1, wherein the user input unit (40) is further configured to receive a guide line from the user, the central processing unit (70) is configured to generate position information of the guide line and the display unit (60) is adapted to display the guide line on the fusion image based on the position information of the guide line.

4. The ultrasound system of Claim 1, wherein the central processing unit (70) is configured to form a guide line based on the position information of the lesion and the position information of the medical needle, and the display unit (60) is adapted to display the guide line on the fusion image based on the position information of the guide line.

5. The ultrasound system of Claim 4, wherein the central processing unit (70) is configured to determine deviation of the medical needle by comparing the position information of the guide line and the position information of the medical needle, and the ultrasound system (110) further comprises a warning unit (61, 62) configured to warn the deviation of the medical needle.

6. The ultrasound system of Claim 1, wherein the central processing unit (70) is configured to determine an arrival time of the medical needle at the lesion based on the position information of the lesion and the position information of the medical needle.

7. The ultrasound system of Claim 1, wherein a wavelength of the first electromagnetic field is different from a wavelength of the second electromagnetic field.

8. The ultrasound system of Claim 1, wherein the image processing unit (50) includes:

> a first image processor (51) configured to form the ultrasound images based on the ultrasound echo signals;

a second image processor (52) configured to reconstruct the external images based on the position information of the probe and the position information of the lesion; and

a third image processor (53) configured to fuse the ultrasound image and the external image received from the first image processor and the second image processor, respectively.

9. The ultrasound system of Claim 8, wherein the second image processor (52) includes:

a coordinate calibration unit (52a) configured to generate coordinates of the lesion in the ultrasound image based on the position information of the probe and calibrating coordinates of the lesion in the external image based on the coordinates of the lesion;

an external image selection unit (52b) configured to select one external image most similar to the ultrasound image among a plurality of external medical images based on the coordinate calibration result; and

an external image reconstruction unit (53c) configured to reconstruct the selected external image.

10. The ultrasound system of Claim 1, wherein the external image signal providing unit (30) is configured to provide the image signals obtained from one of a computerized tomography scanner, a magnetic resonance imaging system and a positron emission tomography scanner.

11. The ultrasound system of Claim 1, wherein the ultrasound echo signals and the position information of the medical needle are inputted in real time.

**Patentansprüche**

1. Ultraschallsystem (100,110,120,130), welches folgendes aufweist:

Eine Sonde (10), welche dafür vorgesehen ist, auf dem Zielobjekt angeordnet zu werden und Ultraschallsignale zu dem Zielobjekt zu übertragen und von dem Zielobjekt reflektierte Ultraschallechosignale zu empfangen, wobei das Zielobjekt eine Verletzung bzw. Wunde aufweist; eine Positionsinformations-Bereitstellungseinheit (20), welche dafür vorgesehen ist, Positionsinformationen der Sonde auf dem Zielobjekt zur Verfügung zu stellen; eine externe medizinische Bildsignal-Bereitstellungseinheit (30), welche dafür vorgesehen ist, externe medizinische Bildsignale des Zielobjekts von einer externen Abbildungsvorrichtung zu empfangen; eine Benutzereingabeeinheit (40), welche dafür vorgesehen ist, Positionsinformationen der Wunde in dem externen medizinischen Bild von einem Benutzer zu empfangen; eine Bildverarbeitungseinrichtung (50), welche dafür vorgesehen ist, ein Ultraschallbild basierend auf den Ultraschallechosignalen zu bilden, das externe Bild basierend auf den externen Bildsignalen zu bilden und ein Fusionsbild des Ultraschallbilds und des externen Bilds basierend auf den Positionsinformationen der Sonde und den Positionsinformationen der Wunde zu bilden; eine Anzeigeeinheit (60), welche dafür vorgesehen ist, das Ultraschallbild, das externe Bild und das Fusionsbild anzuzeigen; und eine zentrale Verarbeitungseinheit (70), welche dafür vorgesehen ist, die Positionsinformations-Bereitstellungseinheit (20), die externe Bildsignal-Bereitstellungseinheit (30), die Benutzereingabeeinheit (40), die Bildverarbeitungseinheit (50) und die Anzeigeeinheit (60) zu steuern, wobei die Positionsinformations-Bereitstellungseinheit (20,80) des weiteren dafür vorgesehen ist, Positionsinformationen einer in das Zielobjekt eingeführten Nadel in dem Ultraschallbild zur Verfügung zu stellen, und wobei die Anzeigeeinheit (60) dafür vorgesehen ist, eine Position der medizinischen Nadel auf dem Fusionsbild basierend auf den Positionsinformationen der medizinischen Nadel unter der Steuerung der zentralen Verarbeitungseinheit anzuzeigen, **dadurch gekennzeichnet, dass** die zentrale Verarbeitungseinheit (70) dafür vorgesehen ist, einen Abstand zwischen der Wunde und der medizinischen Nadel basierend auf den Positionsinformationen der Wunde und den Positionsinformationen der medizinischen Nadel zu berechnen, und die Anzeigeeinheit (30) dafür vorgesehen ist, den Abstand zwischen der Wunde und der medizinischen Nadel auf dem Fusionsbild anzuzeigen, wobei die Positions-Bereitstellungseinheit (20,80) folgendes aufweist:

Einen ersten Feldgenerator (21), welcher dafür vorgesehen ist, ein erstes elektromagnetisches Feld zu erzeugen, um die Position der Sonde zu verfolgen;

einen ersten Detektor (22), der auf der Sonde montiert oder in die Sonde eingebaut ist, um Detektionssignale als Reaktion auf das erste elektromagnetische Feld zu erzeugen;

einen ersten Positionsinformationsgenerator (23), welcher dafür vorgesehen ist, die Positionsinformationen der Sonde zu erzeugen;
einen zweiten Feldgenerator (81), welcher dafür vorgesehen ist, ein zweites elektromagnetisches Feld zu erzeugen, um die Position der medizinischen Nadel zu verfolgen;
einen zweiten Detektor (82), welcher auf der medizinischen Nadel montiert oder in dieselbe eingebaut ist und welcher dafür vorgesehen ist, Detektionssignale als Reaktion auf das zweite elektromagnetische Feld zu erzeugen; und
einen zweiten Positionsinformationsgenerator (83), welcher dafür vorgesehen ist, die Positionsinformationen der medizinischen Nadel zu erzeugen.

2.  Ultraschallsystem nach Anspruch 1, welches des weiteren eine Speichereinheit (90) aufweist, welche dafür vorgesehen ist, das Ultraschallbild, das externe Bild, das Fusionsbild und ein auf der Anzeigeeinheit (60) angezeigtes Bild zu speichern,
    wobei die Benutzereingabeeinheit (40) des weiteren dafür vorgesehen ist, eine Bildschirmspeicheranfrage des Benutzers zu empfangen, die zentrale Verarbeitungseinheit (70) dafür vorgesehen ist, einen auf der Anzeigeeinheit (60) angezeigten Bildschirm als Antwort auf die Bildschirmspeicheranfrage zu erfassen, und wobei der erfasste Bildschirm in der Speichereinheit (90) gespeichert wird.

3.  Ultraschallsystem nach Anspruch 1, wobei die Benutzereingabeeinheit (40) des weiteren dafür vorgesehen ist, eine Richtlinie von dem Benutzer zu empfangen, wobei die zentrale Verarbeitungseinheit (70) dafür vorgesehen ist, Positionsinformationen der Richtlinie zu erzeugen, und wobei die Anzeigeeinheit (60) dafür vorgesehen ist, die Richtlinie auf dem Fusionsbild basierend auf den Positionsinformationen der Richtlinie anzuzeigen.

4.  Ultraschallsystem nach Anspruch 1, wobei die zentrale Verarbeitungseinheit (70) dafür vorgesehen ist, eine Richtlinie basierend auf den Positionsinformationen der Wunde und den Positionsinformationen der medizinischen Nadel zu bilden, und wobei die Anzeigeeinheit (60) dafür vorgesehen ist, die Richtlinie auf dem Fusionsbild basierend auf den Positionsinformationen der Richtlinie anzuzeigen.

5.  Ultraschallsystem nach Anspruch 4, wobei die zentrale Verarbeitungseinheit (70) dafür vorgesehen ist, die Abweichung der medizinischen Nadel durch Vergleichen der Positionsinformationen der Richtlinie und der Positionsinformationen der medizinische Nadel zu ermitteln, und wobei das Ultraschallsystem (110) des weiteren eine Warneinheit (61,62) aufweist, welche dafür vorgesehen ist, bezüglich der Abweichung der medizinischen Nadel zu warnen.

6.  Ultraschallsystem nach Anspruch 1, wobei die zentrale Verarbeitungseinheit (70) dafür vorgesehen ist, einen Ankunftszeitpunkt der medizinischen Nadel an der Wunde basierend auf den Positionsinformationen der Wunde und den Positionsinformationen der medizinischen Nadel zu ermitteln.

7.  Ultraschallsystem nach Anspruch 1, wobei eine Wellenlänge des ersten elektromagnetischen Felds unterschiedlich von einer Wellenlänge des zweiten elektromagnetischen Felds ist.

8.  Ultraschallsystem nach Anspruch 1, wobei die Bildverarbeitungseinheit (50) folgendes aufweist:

    einen ersten Bildprozessor (51), welcher dafür vorgesehen ist, die Ultraschallbilder basierend auf den Ultraschallechosignalen zu bilden;
    einen zweiten Bildprozessor (52), welcher dafür vorgesehen ist, die externen Bilder basierend auf den Positionsinformationen der Sonde und den Positionsinformationen der Wunde zu rekonstruieren; und
    einen dritten Bildprozessor (53), welcher dafür vorgesehen ist, das von dem ersten Bildprozessor bzw. dem zweiten Bildprozessor empfangene Ultraschallbild bzw. externe Bild zu fusionieren.

9.  Ultraschallsignal nach Anspruch (8), wobei der zweite Bildprozessor (52) folgendes aufweist:

    eine Koordinatenkalibrierungseinheit (52a), welche dafür vorgesehen ist, Koordinaten der Wunde in dem Ultraschallbild basierend auf den Positionsinformationen der Sonde zu bilden und Koordinaten der Wunde in dem externen Bild basierend auf den Koordinaten der Wunde zu kalibrieren;
    eine externe Bildauswahleinheit (52b), welche dafür vorgesehen ist,
    ein externes Bild auszuwählen, welches dem Ultraschallbild unter einer Vielzahl von externen medizinischen Bildern basierend auf dem Koordinatenkalibrierungsergebnis am ähnlichsten ist; und
    eine externe Bildrekonstruiereinheit (53c), welche dafür vorgesehen ist, das ausgewählte externe Bild zu rekonstruieren.

**10.** Ultraschallsystem nach Anspruch 1, wobei die externe Bildsignal-Bereitstellungseinheit (30) dafür vorgesehen ist, die von einem computergesteuerten Tomographiescanner, einem magnetischen Resonanzabbildungssystem und einem Positionen-Emissions-Tomographiescanner erlangten Bildsignale zur Verfügung zu stellen.

**11.** Ultraschallsystem nach Anspruch 1,
wobei die Ultraschallechosignale und die Positionsinformationen der medizinischen Nadel in Echtzeit eingegeben werden.

**Revendications**

**1.** Système à ultrasons (100, 110, 120, 130) comprenant une sonde (10) agencée pour être placée sur l'objet cible et pour émettre des signaux ultrasonores vers l'objet cible et recevoir des signaux ultrasonores d'écho réfléchis par l'objet cible, ledit objet cible présentant une lésion; une unité de fourniture d'information de position (20) agencée pour fournir une information de position de la sonde sur l'objet cible ; une unité de fourniture de signaux-image médicaux externes (30) fournissant des signaux-image, agencée pour recevoir des signaux-image médicaux externes de l'objet cible issus d'un dispositif d'imagerie externe; une unité d'entrée de l'utilisateur (40) agencée pour recevoir d'un utilisateur une information de position de la lésion dans l'image médicale externe; une unité de traitement d'image (50) agencée pour former une image ultrasonore sur la base des signaux ultrasonores d'écho, pour former l'image externe sur la base des signaux-image externes et pour former une image de fusion de l'image ultrasonore et de l'image externe sur la base de l'information de position de la sonde et de l'information de position de la lésion; une unité d'affichage (60) agencée pour afficher l'image ultrasonore, l'image externe et l'image de fusion; et une unité de traitement centrale (70) agencée pour commander l'unité de fourniture d'information de position (20), l'unité de fourniture de signaux-image externes (30), l'unité d'entrée de l'utilisateur (40), l'unité de traitement d'image (50) et l'unité d'affichage (60), dans lequel l'unité de fourniture d'information de position (20, 80) est en outre agencée pour fournir, dans l'image ultrasonore, une information de position d'une aiguille médicale introduite dans l'objet cible, et dans lequel l'unité d'affichage (60) est adaptée pour afficher une position de l'aiguille médicale sur l'image de fusion sur la base de l'information de position de l'aiguille médicale sous la commande de l'unité de traitement centrale,
**caractérisé en ce que** l'unité de traitement centrale (70) est agencée pour calculer une distance entre la lésion et l'aiguille médicale sur la base de l'information de position de la lésion et de l'information de position de l'aiguille médicale, et l'unité d'affichage (30) est agencée pour afficher sur l'image de fusion la distance entre la lésion et l'aiguille médicale, dans laquelle l'unité de fourniture d'information de position (20, 80) comprend :

un premier générateur de champ (21) agencé pour générer un premier champ électromagnétique pour poursuivre la position de la sonde;
un premier détecteur (22) monté sur ou incorporé dans la sonde pour générer des signaux de détection en réponse au premier champ électromagnétique;
un premier générateur d'information de position (23) agencé pour générer l'information de position de la sonde ;
un second générateur de champ (81) agencé pour générer un second champ électromagnétique pour poursuivre la position de l'aiguille médicale ;
un second détecteur (82) monté sur ou incorporé dans l'aiguille médicale, agencé pour générer des signaux de détection en réponse au second champ électromagnétique ; et
un second générateur d'information de position (83) agencé pour générer l'information de position de l'aiguille médicale.

**2.** Système ultrasonore de la revendication 1, comprenant en outre une unité de stockage (90) agencée pour stocker l'image ultrasonore, l'image externe, l'image de fusion et une image affichée sur l'unité d'affichage (60).
dans lequel l'unité d'entrée de l'utilisateur (40) est agencée pour recevoir en outre une requête de sauvegarde de l'écran d'affichage en provenance de l'utilisateur, l'unité de traitement centrale (70) est agencée pour capturer un écran affiché sur l'unité d'affichage (60) en réponse à la requête de sauvegarde de l'écran d'affichage et l'écran capturé est stocké dans l'unité de stockage (90).

**3.** Système ultrasonore de la revendication 1, dans lequel l'unité d'entrée de l'utilisateur (40) est en outre agencée pour recevoir une ligne de guidage en provenance de l'utilisateur, l'unité de traitement centrale (70) est agencée pour générer une information de position de la ligne de guidage et l'unité d'affichage (60) est agencée pour afficher la ligne de guidage sur l'image de fusion sur la base de l'information de position de la ligne de guidage.

**4.** Système ultrasonore de la revendication 1, dans lequel l'unité de traitement centrale (70) est agencée pour former une ligne de guidage sur la base de l'information de position de la lésion et de l'information de position de l'aiguille médicale, et l'unité d'affichage (60) est agencée pour afficher la ligne de guidage sur l'image de fusion sur la base de l'information de position de la ligne de guidage.

**5.** Système ultrasonore de la revendication 4, dans lequel l'unité de traitement centrale (70) est agencée pour déterminer la déviation de l'aiguille médicale en comparant l'information de position de la ligne de guidage et l'information de position de l'aiguille médicale, et le système ultrasonore (110) comprend en outre une unité d'alarme (61, 62) agencée pour avertir de la déviation de l'aiguille médicale.

**6.** Système ultrasonore de la revendication 1, dans lequel l'unité de traitement centrale (70) est agencée pour déterminer un temps d'arrivée de l'aiguille médical à la lésion sur la base de l'information de position de la lésion et de l'information de position de l'aiguille médicale.

**7.** Système ultrasonore de la revendication 1, dans lequel une longueur d'onde du premier champ électromagnétique est différente d'une longueur d'onde du second champ électromagnétique.

**8.** Système ultrasonore de la revendication 1, dans lequel l'unité de traitement d'image (50) comprend :

un premier processeur d'image (51) agencé pour former les images ultrasonores sur la base des signaux ultrasonores d'écho ;
un deuxième processeur d'image (52) agencé pour reconstruire les images externes sur la base de l'information de position de la sonde et de l'information de position de la lésion, et
un troisième processeur d'image (53) agencé pour fusionner l'image ultrasonore et l'image externe reçues respectivement en provenance du premier processeur d'image et du deuxième processeur d'image.

**9.** Système ultrasonore de la revendication 8, dans lequel le deuxième processeur d'image (52) comprend :

une unité d'étalonnage de coordonnées (52a) agencée pour générer des coordonnées de la lésion dans l'image ultrasonore sur la base de l'information de position de la sonde et pour étalonner les coordonnées de la lésion dans l'image externe sur la base des coordonnées de la lésion;
une unité de sélection d'image externe (52b) agencée pour sélectionner une image externe plus semblable à l'image ultrasonore parmi une pluralité d'images médicales externes sur la base du résultat de l'étalonnage des coordonnées ; et
une unité de reconstruction d'image externe (53c) agencée pour reconstruire l'image externe sélectionnée.

**10.** Système ultrasonore de la revendication 1, dans lequel l'unité de fourniture de signaux-image externes (30) est agencée pour fournir les signaux-image obtenus d'un scanner tomographique informatisé, d'un système d'imagerie par résonance magnétique et d'un scanner tomographique à émission de positrons.

**11.** Système ultrasonore de la revendication 1, dans lequel les signaux ultrasonores d'écho et l'information de position de l'aiguille médicale sont entrés en temps réel.

FIG. 1

100

10 PROBE

20 PROBE POSITION INFORMATION PROVIDING UNIT

70 CENTRAL PROCESSING UNIT

60 DISPLAY UNIT

40 USER INPUT UNIT

30 EXTERNAL IMAGE SIGNAL PROVIDING UNIT

50 IMAGE PROCESSING UNIT

FIG. 2

POSITION MARKER

TARGET OBJECT

LESION

FIG. 3

# FIG. 4

EP 1 913 875 B1

FIG. 5

# FIG. 6

130

10 — PROBE

20 — PROBE POSITION INFORMATION PROVIDING UNIT

80 — MEDICAL NEEDLE POSITION INFORMATION PROVIDING UNIT

62 — SECOND WARNING UNIT

61 — FIRST WARNING UNIT

50 — IMAGE PROCESSING UNIT

70 — CENTRAL PROCESSING UNIT

60 — DISPLAY UNIT

30 — EXTERNAL IMAGE SIGNAL PROVIDING UNIT

40 — USER INPUT UNIT

90 — STORING UNIT

EP 1 913 875 B1

## FIG. 7

```
                    ┌─────────────────────┐
                    │      CENTRAL        │
                    │  PROCESSING UNIT    │──── 70
                    └─────────────────────┘
                                                              20
  ┌──────────────────────────────────────────────────────────────┐
  │   ┌─────────────────┐ ─── 21                                  │
  │   │   FIRST FIELD   │                                         │
  │   │   GENERATOR     │                                         │
  │   └─────────────────┘                                         │
  │              22                  23                           │
  │   ┌─────────────────┐   ┌──────────────────────────┐         │
  │   │     FIRST       │   │    FIRST POSITION        │         │
  │   │   DETECTOR      │──▶│ INFORMATION GENERATOR    │         │
  │   └─────────────────┘   └──────────────────────────┘         │
  └──────────────────────────────────────────────────────────────┘
```

## FIG. 8

```
                    ┌─────────────────────┐
                    │      CENTRAL        │
                    │  PROCESSING UNIT    │──── 70
                    └─────────────────────┘
                                                              80
  ┌──────────────────────────────────────────────────────────────┐
  │   ┌─────────────────┐ ─── 81                                  │
  │   │  SECOND FIELD   │                                         │
  │   │   GENERATOR     │                                         │
  │   └─────────────────┘                                         │
  │              82                  83                           │
  │   ┌─────────────────┐   ┌──────────────────────────┐         │
  │   │    SECOND       │   │    SECOND POSITION       │         │
  │   │   DETECTOR      │──▶│ INFORMATION GENERATOR    │         │
  │   └─────────────────┘   └──────────────────────────┘         │
  └──────────────────────────────────────────────────────────────┘
```

## FIG. 9

## FIG. 10

## FIG. 11

EP 1 913 875 B1

**EP 1 913 875 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2005033160 A1
- US 6546279 B
- WO 2006008300 A